# EUROPÄISCHE PATENTSCHRIFT

(11) **EP 2 937 144 B1**
(45) Veröffentlichungstag und Bekanntmachung des Hinweises auf die Patenterteilung: **07.03.2018**
(21) Anmeldenummer: 15163503.4
(22) Anmeldetag: 14.04.2015
(51) Int. Cl.: C12M 1/12, C12N 1/06, C12M 3/06, C12M 1/00, B01L 3/00

(54) **VERFAHREN UND MIKROFLUIDISCHES SYSTEM ZUM AUFBEREITEN VON ORGANISCHEN ZELLEN UND HERSTELLUNGSVERFAHREN ZUM HERSTELLEN EINES MIKROFLUIDISCHEN SYSTEMS ZUM AUFBEREITEN VON ORGANISCHEN ZELLEN**
METHOD AND MICROFLUIDIC SYSTEM FOR THE TREATMENT OF ORGANIC CELLS AND A METHOD FOR PRODUCING FOR THE PRODUCTION OF A MICROFLUIDIC SYSTEM FOR THE TREATMENT OF ORGANIC CELLS
PROCÉDÉ ET SYSTÈME MICROFLUIDIQUE DESTINÉ À LA PRÉPARATION DE CELLULES ORGANIQUES ET PROCÉDÉ DE PRODUCTION D'UN SYSTÈME MICROFLUIDIQUE DESTINÉ À LA PRÉPARATION DE CELLULES ORGANIQUES

(30) Priorität: 25.04.2014 DE 102014207775
(43) Veröffentlichungstag der Anmeldung: 28.10.2015
(73) Patentinhaber: ROBERT BOSCH GMBH, 70442 Stuttgart (DE)
(72) Erfinder: Faltin, Bernd, 70839 Gerlingen (DE); Dorrer, Christian, 70193 Stuttgart (DE)

(56) Entgegenhaltungen:
- EP-A2- 2 026 074
- WO-A1-99/33559
- US-A1- 2008 057 505
- US-A1- 2010 055 766
- US-A1- 2010 256 350

## Beschreibung

### Stand der Technik

Die vorliegende Erfindung bezieht sich auf ein Verfahren zum Aufbereiten von organischen Zellen auf ein mikrofluidisches System zum Aufbereiten von organischen Zellen sowie auf ein Herstellungsverfahren zum Herstellen eines derartigen mikrofluidischen Systems.

In der molekularen Diagnostik besteht oftmals die Notwendigkeit zum Nachweis von pathogenen Nukleinsäuren wie DNA oder RNA aus einer Probe. Als pathogene DNA oder RNA wird die aus einem Pathogen, z. B. einem Virus, einem Bakterium oder einem Pilz, gewonnene DNA oder RNA bezeichnet. Als Probe wird die zu analysierende Flüssigkeit, typischerweise eine flüssige oder verflüssigte Patientenprobe, z. B. Blut, Urin, Stuhl, Sputum, Liquor, Lavage, ein ausgespülter Abstrich oder eine verflüssigte Gewebeprobe, bezeichnet. Die Nukleinsäuren werden aufgereinigt und einer Analyse zugeführt, durch die das Vorhandensein bestimmter Pathogene oder Gene, z. B. Resistenzgene, überprüft wird. Diese Analyse kann z. B. durch Sequenzierung, Polymerase-Kettenreaktion (Polymerase Chain Reaction; PCR), Real-Time-PCR und/oder Hybridisierung auf einem Microarray erfolgen.

Die Aufreinigung einer Nukleinsäure aus einer Probe erfolgt oftmals durch Aufschließen bzw. Lyse der Pathogene und anschließende Adsorption der Nukleinsäuren an einer festen Phase, z. B. einem Silikafilter oder Mikropartikeln in Form von Kügelchen, sogenannten Beads. Neben chemischen und enzymatischen Methoden zur Lyse gibt es dabei auch mechanische Methoden, z. B. mit Ultraschall oder durch Zermahlen mit Beads. Ziel der Aufreinigung ist es, die Nukleinsäure in aufkonzentrierter Form einer darauffolgenden Amplifikation und/oder Detektion zur Verfügung zu stellen. Um die Pathogene vor der Aufreinigung aufzukonzentrieren, wird die Probe z. B. zentrifugiert oder über einen Filter gespült.

Die EP 1179 585 B1 beschreibt eine Kartusche zum Lysieren von Komponenten einer Fluid-Probe. Die Kartusche weist eine Lysierkammer mit einer Wand auf, an welche ein Ultraschalltransducer koppelbar ist, um eine Übertragung von Ultraschallenergie in die Lysierkammer zu bewirken.

Aus den Dokumenten US 2010/0055766 A1, WO 99/33559, US 2010/0256350 A1, US 2008/0058505 und EP 2026074 A2 sind mikrofluidische Vorrichtungen zur Separierung von Analyten aus biologischen Proben bekannt.

### Offenbarung der Erfindung

Vor diesem Hintergrund wird mit dem hier vorgestellten Ansatz ein Verfahren zum Aufbereiten von organischen Zellenvorgestellt. Vorteilhafte Ausgestaltungen ergeben sich aus den jeweiligen Unteransprüchen und der nachfolgenden Beschreibung.

Eine Mehrzahl von zu einer Schüttung angehäuften Mikropartikeln kann zur Akkumulation von organischen Zellen und basierend auf einer anschließenden Resuspendierung in einem Lysepuffer zur Lyse der Zellen, gegebenenfalls mit zusätzlicher mechanischer Agitation, eingesetzt werden. Zusätzlich können die Mikropartikel auch zur Aufreinigung einer aus den organischen Zellen freigesetzten Substanz, z. B. einer DNA, genutzt werden.

Bisher bekannte Verfahren verwenden Zentrifugation oder Gewebefilter bzw. Membranen zur Akkumulation von Zellen aus einer Probe. Eine Zentrifugation bietet den Nachteil, dass sie nicht in einem mikrofluidischen System umsetzbar ist und deshalb schwer in einen automatisierten Ablauf eingebunden werden kann. Bei der Verwendung eines Gewebefilters bzw. einer Membran ist zwar eine Integration in ein mikrofluidisches System bzw. eine anderweitige Automatisierung möglich, jedoch sind die auf dem Gewebefilter akkumulierten Zellen dann in diesem sozusagen "versteckt" und für nachfolgende Prozessierungsschritte, z. B. eine Lyse, schwerer bzw. mit schlechterer Effizienz zu erreichen. Die Erfindung löst diese Probleme durch das reversible Packen zu einem Filterelement und Resuspendieren von Mikropartikeln. Hierdurch können zwei entgegengesetzte Anforderungen, nämlich das Bereitstellen eines Elements mit kleinen Poren beispielsweise zur Akkumulation von Zellen und zum Binden von Nukleinsäuren und das möglichst effiziente und vollständige Umspülen beispielsweise von akkumulierten Zellen oder gebundenen Nukleinsäuren mit Reagenzien, z.B. Lyse- oder Waschreagenzien, mit nur einem Bauteil realisiert werden.

Gemäß dem hier vorgeschlagenen Konzept ermöglicht die Akkumulation von Zellen auf einer Mikropartikel-Schüttung eine für eine spätere Analyse vorteilhafte Aufkonzentrierung der Zellen. Denn es kann auf diese Weise eine Sensitivität einer sich gegebenenfalls anschließenden Detektion von z. B. Pathogenen in den Zellen verbessert werden. Unter Umständen können auf diese Weise geringe Zellzahlen in einem großen Volumen überhaupt erst detektiert werden. Durch die Resuspendierung der Mikropartikel werden die Zellen in einem kleineren Volumen wieder freigesetzt und können vorteilhaft effektiv lysiert werden, wodurch sich ein Aufkonzentrationseffekt ergibt.

Als weiterer Vorteil kann durch die Verwendung der Mikropartikel für Akkumulation und Aufreinigung auf eine Membran bzw. einen weiteren Filter für die Aufreinigung verzichtet werden. Ein erfindungsgemäßes System kann dadurch kompakter und kostengünstiger gestaltet werden. In einer Weiterbildung des hier vorgeschlagenen Ansatzes kann mittels mechanischer Agitation der Mikropartikel während der Lyse eine Mahlwirkung erzielt und so auch schwierig zu lysierende Zellen, z. B. Pilze, lysiert werden.

Ein hier vorgeschlagenes Aufbereitungsverfahren zur Analyse organischer Zellen eignet sich insbesondere für eine Integration in ein mikrofluidisches System. Der Verfahrensablauf wird somit der Automatisierung zugänglich, wodurch Zeit und Kosten eingespart werden können. Weiterhin bietet eine Automatisierung in einem mikrofluidischen System den Vorteil, dass das System ohne Spezialkenntnisse bedient werden kann und die Gefahr von Kontaminationen verringert wird.

Es wird ein Verfahren zum Aufbereiten von organischen Zellen vorgestellt, wobei das Verfahren die folgenden Schritte aufweist:
Bereitstellen eines mikrofluidischen Systems mit einer eine stationäre Phase in Form einer Mehrzahl von Mikropartikeln aufweisenden Kammer;
Einlassen einer Mehrzahl von organischen Zellen in einer mobilen Phase in die Kammer über eine erste Öffnung des mikrofluidischen Systems;
Akkumulieren der organischen Zellen in einem (insbesondere verjüngten) Abschnitt der Kammer, der einem zumindest für die Mikropartikel (in einer Variante auch für die organischen Zellen) undurchlässigen Filter des mikrofluidischen Systems vorgeschaltet ist; und
Einspülen eines Lysemittels in die Kammer, um für eine Aufschließung der organischen Zellen die Mikropartikel und die organischen Zellen in der Kammer zu resuspendieren.

Der Filter des mikrofluidischen Systems ist dabei beispielsweise zwischen einer zweiten Öffnung und dem (insbesondere sich verjüngenden) Abschnitt angeordnet. Insofern ist der Filter in einer Flussrichtung von Fluid aus der zweiten Öffnung über den sich verjüngenden Abschnitt diesem sich verjüngenden Abschnitt vorgeschaltet. Anders ausgedrückt ist der Filter bei einem Einfluss von Fluid über die erste Öffnung und den sich verjüngenden Abschnitt der Kammer dem sich verjüngenden Abschnitt nachgelagert. Das Filter kann ferner für organische Zellen in einer Ausführungsform durchlässig und in einer anderen Ausführungsform undurchlässig sein.

Mittels des Verfahrens können die Zellen in dem Sinne aufbereitet werden, dass in den Zellen enthaltene Nukleinsäuren wie DNA oder RNA einer Analyse zugänglich gemacht werden können. Bei dem mikrofluidischen System kann es sich um einen Schichtaufbau mit mikro- bis millimetergroßen Strukturen zur Analyse der organischen Zellen, beispielsweise um ein sogenanntes Lab-on-a-Chip-System, handeln. Die organischen Zellen können dabei in Form einer Probe in einem Fluid, z. B. einem Körperfluid, enthalten sein, und damit in der Kammer so mit der stationären Phase in Wechselwirkung gebracht werden, dass mithilfe des Lysemittels die Zellen aufgespalten bzw. lysiert werden und dabei die Nukleinsäuren freigesetzt werden. Zu diesem Zweck kann in einer Variante des hier vorgestellten Ansatzes die stationäre Phase in Form der Mikropartikel als ein "Werkzeug" zum mechanischen Aufbrechen der Zellen verstanden werden. Unter dem Resuspendieren kann ein Aufwirbeln oder Verwirbeln der organischen Zellen und der Mikropartikel innerhalb der Kammer verstanden werden.

Gemäß einer Ausführungsform des Verfahrens kann in dem Schritt des Einspülens das Lysemittel derart eingespült werden, dass die Mikropartikel und die organischen Zellen in der Kammer resuspendiert werden. Dies kann beispielsweise erfolgen, indem das Lysemittel über die dem Filter nachgeschaltete zweite Öffnung des mikrofluidischen Systems in die Kammer eingespült wird. So kann die Resuspendierung, also die Aufwirbelung, der Zellen und Mikropartikel und damit das Aufbrechen der Zellen vorteilhaft unterstützt und verbessert werden.

Alternativ oder ergänzend zum Resuspendieren der Mikropartikel durch Strömungskräfte kann das Resuspendieren der Mikropartikel durch Einkoppeln von weiterer mechanischer Energie, z.B. Ultraschall oder Stoßwellen, in die Kammer unterstützt werden. Dies hat den Vorteil, dass die mechanische Agitation der Mikropartikel und somit die Lysewirkung verstärkt wird.

Gemäß einer weiteren Ausführungsform kann das Verfahren einen Schritt des Einfüllens bzw. Einspülens der Mikropartikel über die Einlassöffnung in die Kammer und/oder des Packens der Mikropartikel in dem verjüngten Abschnitt der Kammer aufweisen. Dies hat den Vorteil, dass eine besonders dichte Packung der Mikropartikel erreicht werden kann, was die Effizienz der Akkumulation der organischen Zellen erhöht. Insbesondere kann so z. B. nach einem Transport des Systems, bei dem sich ggf. durch einwirkende Stöße oder Vibrationen die Packung der Mikropartikel gelockert hat, eine erneute dichte Packung der Mikropartikel erreicht werden. Ein weiterer Vorteil ist, dass die Kammer in vorhergehenden Prozessschritten für andere Zwecke genutzt werden kann, z. B. als Reaktions- oder Vorlagerungskammer. Die Mikropartikel werden dann erst bei Bedarf eingespült. Weiterhin können so ein die Kammer aufweisendes Element des mikrofluidischen Systems und die stationäre Phase unabhängig voneinander bereitgestellt werden und damit ein Anwendungsfeld des mikrofluidischen Systems vorteilhaft erweitert werden, indem das mikrofluidische System z. B. kundenspezifisch ausgerichtet und angepasst werden kann. Das Einfüllen bzw. Einspülen der Mikropartikeln kann z. B. erfolgen, indem die Mikropartikel in einer mobilen Phase, z. B. Wasser oder einem wässrigen Puffer, in die Kammer gepumpt oder pipettiert werden. Das Packen der Mikropartikel kann z. B. durch Sedimentieren durch Schwerkraft oder durch Pumpen oder Spülen einer Flüssigkeit über die Mikropartikel erfolgen, wobei die Flussrichtung dabei von der ersten Öffnung der Kammer über den (insbesondere sich verjüngenden) Abschnitt und den Filter in die zweite Öffnung gerichtet ist. Wird eine Pumpe verwendet, so kann diese sich stromauf- oder stromabwärts der Kammer befinden, so dass die Flüssigkeit entweder mit Überdruck über die Mikropartikel gepumpt oder mit Unterdruck gesaugt wird.

Ferner kann das Verfahren einen Schritt des Einbringens eines Bindemittels, z. B. eines Bindepuffers, in die Kammer aufweisen. Damit können basierend auf der Aufschließung aus den Zellen freigesetzte Nukleinsäuren an die Mikropartikel gebunden werden, z. B. aufgrund elektrostatischer Interaktionen. Mit dieser Ausführungsform des Verfahrens kann eine Weiterbehandlung der freigesetzten Nukleinsäuren, wie z. B. eine Waschung oder eine Akkumulation auf den Mikropartikeln, ohne Weiteres vorbereitet werden.

Weiterhin kann das Verfahren einen Schritt des Packens der Mikropartikel mit den an die Mikropartikel angebundenen Nukleinsäuren in dem verjüngten Abschnitt der Kammer aufweisen. Mit dieser Ausführungsform kann eine Aufkonzentration der Nukleinsäuren auf möglichst kleinem Raum und damit eine Eluierung der Nukleinsäuren aus einem möglichst kleinen Volumen durchgeführt werden. Das Packen kann in diesem Schritt z. B. geschehen, indem die in der Kammer vorhandene Flüssigkeit bzw. das Bindemittel durch die zweite Öffnung abgesaugt wird.

Um eine besonders gute Reinheit der Nukleinsäuren zu erreichen, kann im Anschluss an den Schritt des Einbringens des Bindemittels eine Waschung der Mikropartikel durchgeführt werden. Eine Waschung kann z. B. erfolgen, indem ein Waschpuffer über die Mikropartikel geleitet wird. Auch in diesem Schritt kann der Waschpuffer so in die Kammer geleitet werden, dass eine Resuspendierung der Mikropartikel erfolgt, z. B. durch Einspülen über die zweite Öffnung. Dies hat den Vorteil, dass Kontaminationen besonders effektiv herausgespült werden.

Das Verfahren kann einen Schritt des Eluierens der Nukleinsäuren von den Mikropartikeln und des Transportierens der Nukleinsäuren durch den Filter und die zweite Öffnung aus der Kammer aufweisen. Dieser Schritt kann ausgeführt werden, indem z. B. durch die erste oder zweite Öffnung Wasser und/oder ein Elutionsmittel über die vor dem Filter akkumulierten Nukleinsäuren gespült wird. Beispielsweise kann das Elutionsmittel über die zweite Öffnung in die Kammer eingespült, für eine gewisse Zeitspanne inkubiert und dann durch die zweite Öffnung wieder abgesaugt werden. Diese Ausführungsform gewährleistet auf schnelle und einfache Weise eine Trennung der untersuchten Substanz von den Mitteln zu ihrer Verfügbarkeitsmachung. Gegebenenfalls kann die Kammer während des Eluierens auch beheizt werden, z.B. auf Temperaturen zwischen 30 und 70 °C.

Das Verfahren kann vor dem Schritt des Eluierens der Nukleinsäuren einen Schritt der Trocknung der Mikropartikel und des Filters aufweisen. Dies hat den Vorteil, dass Reste des Bindemittels besonders effizient entfernt werden und somit eine vollständigere Elution möglich ist. Die Trocknung kann z.B. durch Spülen von Luft oder Stickstoff über die Mikropartikel und/oder durch Heizen der Kammer, z.B. auf Temperaturen zwischen 40 und 70 °C, erfolgen.

Gemäß einer weiteren Ausführungsform kann das Verfahren einen Schritt des Einbringens eines Reinigungsmittels, z. B. eines Waschpuffers, in die Kammer aufweisen, um die organischen Zellen zu reinigen. Dieser Schritt kann nach dem Schritt des Akkumulierens der organischen Zellen in dem verjüngten Abschnitt der Kammer durchgeführt werden. Dieser Verfahrensschritt verbessert allgemein die Reinheit der Probe, insbesondere werden nachfolgende Prozessschritte störende Substanzen entfernt. Das Einbringen des Reinigungsmittels erfolgt in diesem Fall vorteilhafterweise durch die erste Öffnung, um die Packung der Mikropartikel nicht zu stören und zu vermeiden, dass akkumulierte Zellen aus der Kammer ausgespült werden und somit verloren gehen.

Ferner wird ein mikrofluidisches System zum Aufbereiten von organischen Zellen vorgestellt, wobei das mikrofluidische System die folgenden Merkmale aufweist:
eine Kammer zum Aufnehmen einer in Form einer Mehrzahl von Mikropartikeln vorliegenden stationären Phase und einer eine Mehrzahl von organischen Zellen aufweisenden mobilen Phase, wobei die Kammer einen für eine Akkumulation der Mikropartikel und/oder der organischen Zellen geeigneten (insbesondere verjüngten) Abschnitt aufweist;
eine mit der Kammer gekoppelte erste Öffnung zum Einlassen der Mikropartikel und/oder der organischen Zellen in die Kammer;
ein dem (insbesondere verjüngten) Abschnitt der Kammer nachgeschalteter, zumindest für die Mikropartikel undurchlässiger, Filter; und
eine dem Filter nachgeschaltete, mit der Kammer gekoppelte, zweite Öffnung.

Unter dem mikrofluidischen System kann eine Vorrichtung verstanden werden, die unter Verwendung von Flüssigkeiten und/oder Gasen auf kleinstem Raum betrieben wird. Die Kammer kann eine längliche Form aufweisen, wobei die erste Öffnung an einer kurzen Seite der Kammer angeordnet sein kann. Bei dem verjüngten Abschnitt der Kammer kann es sich um einen Bereich der wannenförmigen Kammer mit einer geringeren Tiefe als ein Hauptabschnitt der wannenförmigen Kammer handeln. Der verjüngte Abstand kann in maximaler Entfernung von der ersten Öffnung ausgebildet sein. Die erste Öffnung kann generell als eine Einlassöffnung in die Kammer und die zweite Öffnung generell als eine Auslassöffnung aus der Kammer bezeichnet werden. Die Nachschaltung des Filters nach dem verjüngten Abschnitt und die Nachschaltung der zweiten Öffnung nach dem Filter kann als in Bezug zu einer Position der Kammer gesetzt verstanden werden. Auch kann die Nachschaltung des Filters nach dem verjüngten Abschnitt und die Nachschaltung der zweiten Öffnung nach dem Filter als in Bezug zu einer grundsätzlichen Funktion des mikrofluidischen Systems verstanden werden, gemäß der eine zu untersuchende Substanz zu Beginn des oben beschriebenen Verfahrens des Aufbereitens von organischen Zellen über die erste Öffnung dem mikrofluidischen System zugeführt wird und am Ende des Verfahrens über die zweite Öffnung dem mikrofluidischen System entnommen wird. Der verjüngte Abschnitt sowie die zweite Öffnung der Kammer können sich bzgl. der Gravitation am unteren Ende der Kammer befinden, so dass die Mikropartikel in Abwesenheit einer Strömung im verjüngten Abschnitt sedimentieren. Die erste Öffnung kann sich am bzgl. der Gravitation oberen Ende der Kammer befinden. Dies hat den Vorteil, dass bei einem Einspülen von Flüssigkeiten in die Kammer über die zweite Öffnung in der Kammer enthaltene Luft über die erste Öffnung entweichen kann. Diese Belüftungsfunktion kann in einer Variante des Systems auch von einer weiteren, dritten Öffnung wahrgenommen werden. Vorteilhafterweise ist der dem Filter gegenüberliegende und zur Aufnahme der Packung von Mikropartikeln vorgesehene Bereich der Kammer so gestaltet, dass keine fluidisch "toten" Ecken entstehen, d.h. Bereiche, die bei dem Spülen von Flüssigkeiten über die Mikropartikel nicht oder nur schlecht durchströmt werden. Hierzu kann das bzgl. der Gravitation unten liegende Ende der Kammer schräg bzw. abgerundet zum Filter hin verlaufen. Insbesondere kann dabei der Boden der Kammer der Form des Filters so angepasst sein, dass kein Teilbereich der Kammer bzgl. der Graviation tiefer als das unterste Ende des Filters liegt.

Gemäß einer Ausführungsform kann eine Wandung der Kammer des mikrofluidischen Systems bzw. ein Teilbereich derselben, z. B. ein kreisförmiger Teilbereich, elastisch, insbesondere als eine Membran, ausgebildet sein. Damit eignet sich die Wandung zur Übertragung von Stoßwellen oder Ultraschallimpulsen an einen Inhalt der Kammer. Somit kann auf einfache Weise durch zusätzliche mechanische Agitation eine zusätzliche Mahl- und/oder Lysewirkung auf die organischen Zellen ausgeübt werden.

Insbesondere kann das mikrofluidische System als ein Schichtsystem aus zumindest einem Bodenelement und einem Deckelelement zum Abdecken des Bodenelements ausgebildet sein. Dabei kann die Kammer wannenförmig in dem Bodenelement angelegt sein und die erste Öffnung Teil eines das Bodenelement durchgreifenden ersten Kanals sein und der Filter in einem dem verjüngten Abschnitt der Kammer gegenüberliegenden Bereich des Deckelelements angeordnet sein. In dieser modularen Form kann das mikrofluidische System besonders schnell und kostengünstig hergestellt und ohne Weiteres gemäß Kundenwünschen angepasst werden.

Gemäß einer Ausführungsform kann der Filter an einer der Kammer zugewandten Hauptseite des Deckelelements angeordnet sein und die zweite Öffnung Teil eines an den Filter angrenzenden, das Deckelelement durchgreifenden, zweiten Kanals sein. In dieser Ausführungsform kann das mikrofluidische System vorteilhaft mit kleinem Bauraum realisiert werden.

Alternativ kann das mikrofluidische System dadurch gekennzeichnet sein, dass der Filter an einer von der Kammer abgewandten weiteren Hauptseite des Deckelelements angeordnet und über einen in dem Deckelelement angelegten Verbindungskanal mit der Kammer gekoppelt ist. Die zweite Öffnung kann dann Teil eines an den Filter angrenzenden zweiten Kanals sein, der ein das Deckelelement abdeckendes Abdeckelement durchgreift. Diese Ausführungsform gewährleistet ein vorteilhaftes gleichmäßigeres Anströmen der zu untersuchenden Substanzen an den Filter.

Ferner kann die Kammer in eine mit der ersten Öffnung gekoppelte Lysekammer und eine den verjüngten Abschnitt aufweisende Filterkammer unterteilt sein. Die Lysekammer und die Filterkammer können über einen Durchtrittskanal verbunden sein. Diese Ausführungsform weist den Vorteil auf, dass die Filterung der zu untersuchenden Substanzen in weiterer Entfernung von der Lysierung der sie enthaltenden organischen Zellen durchgeführt werden kann, womit eine Konzentration der zu untersuchenden Substanzen vorteilhaft maximiert werden kann.

Gemäß einer weiteren Ausführungsform des mikrofluidischen Systems kann die Kammer eine Röhrenform aufweisen. Dabei kann der Filter einen Boden der Kammer bilden. Auch diese Ausführungsform kann eine homogenere Anströmung des Filters durch die zu untersuchenden Substanzen ermöglichen.

Es wird ferner ein Herstellungsverfahren zum Herstellen eines mikrofluidischen Systems zum Aufbereiten von organischen Zellen vorgestellt, wobei das Herstellungsverfahren die folgenden Schritte aufweist:
Bereitstellen eines Bodenelements, in dem eine Kammer wannenförmig angelegt ist und das eine erste Öffnung aufweist, die Teil eines das Bodenelement durchgreifenden ersten Kanals ist;
Bereitstellen eines Deckelelements;
Bereitstellen eines Filters im Boden- oder Deckelelement; und
Aufsetzen des Deckelelements auf dem Bodenelement so, dass der Filter einem (insbesondere verjüngten) Abschnitt der Kammer benachbart angeordnet wird.

Auch durch diese Ausführungsvariante der Erfindung in Form eines Herstellungsverfahrens kann die der Erfindung zugrunde liegende Aufgabe schnell und effizient gelöst werden.

Der hier vorgestellte Ansatz wird nachstehend anhand der beigefügten Zeichnungen beispielhaft näher erläutert. Es zeigen:
Fig. 1 ein Blockschaltbild einer mikrofluidischen Systems zum Aufbereiten von organischen Zellen, gemäß einem Ausführungsbeispiel der vorliegenden Erfindung;
Fig. 2 eine Schnittdarstellung eines mikrofluidischen Systems gemäß einem Ausführungsbeispiel der vorliegenden Erfindung;
Fig. 3 eine weitere Schnittdarstellung des mikrofluidischen Systems aus Fig. 2;
Fig. 4 eine Schnittdarstellung eines mikrofluidischen Systems gemäß einem weiteren Ausführungsbeispiel der vorliegenden Erfindung;
Fig. 5 ein Ablaufdiagramm eines Verfahrens zum Aufbereiten von organischen Zellen, gemäß einem Ausführungsbeispiel der vorliegenden Erfindung;
Fig. 6 bis 11 Phasenbilder zur Erläuterung einer Funktionsweise des mikrofluidischen Systems aus Fig. 2, gemäß einem Ausführungsbeispiel der vorliegenden Erfindung;
Fig. 12 ein Ablaufdiagramm eines Herstellungsverfahrens zum Herstellen eines mikrofluidischen Systems zum Aufbereiten von organischen Zellen, gemäß einem Ausführungsbeispiel der vorliegenden Erfindung.
Fig. 13 ein Blockschaltbild eines mikrofluidischen Systems gemäß einem Ausführungsbeispiel der vorliegenden Erfindung;
Fig. 14 ein Blockschaltbild eines weiteren mikrofluidischen Systems gemäß einem Ausführungsbeispiel der vorliegenden Erfindung;
Fig. 15 Teilfiguren von Komponenten eines mikrofluidischen Systems gemäß einem Ausführungsbeispiel der vorliegenden Erfindung in unterschiedliche Ansichten; und
Fig. 16 Teilfiguren von weiteren Komponenten eines mikrofluidischen Systems gemäß einem Ausführungsbeispiel der vorliegenden Erfindung in unterschiedliche Ansichten.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

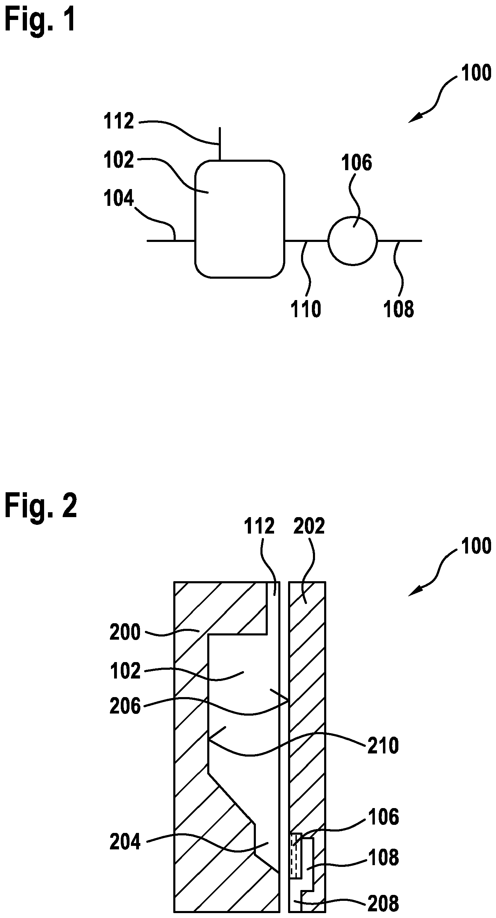

Fig. 1 zeigt ein Blockschaltbild eines Ausführungsbeispiels eines mikrofluidischen Systems 100 zum Aufbereiten von organischen Zellen. Das mikrofluidische System 100 wird zur Aufbereitung von organischen Zellen unter Verwendung von Mikropartikeln eingesetzt und umfasst eine Kammer 102, einen ersten Kanal 104, ein Filter 106 und einen zweiten Kanal 108. Der erste Kanal 104 bildet dabei einen Einlasskanal zum Einlassen von Mikropartikeln und/oder einer Probe mit organischen Zellen in die Kammer 102. Der zweite Kanal 108 kontaktiert eine Rückseite des Filters bzw. Filterelements 106 und bildet einen Auslasskanal zum Auslassen einer mithilfe der Mikropartikel und des Lysemittels aus den organischen Zellen herausgebrochenen und mittels der Packung von Mikropartikeln aufgereinigten Substanz aus dem mikrofluidischen System 100. Über einen Verbindungskanal 110 sind die Kammer 102 und das Filterelement 106 fluidisch verbunden, wobei der Verbindungskanal auch entfallen bzw. ein Teil der Kammer sein kann. Bei dem in Fig. 1 gezeigten Ausführungsbeispiel ist das mikrofluidische System 100 zusätzlich mit einem Belüftungskanal 112 zum Belüften der Kammer 102 ausgestattet. Ein Volumen der Kammer 102, in der Lyse und Aufreinigung der Probe stattfinden, liegt zwischen z. B. 100 Mikrolitern und 10 Millilitern, typischerweise bei etwa zwei Millilitern. Das Filterelement 106 liegt beispielsweise in Form einer Kunststofffritte, eines porösen Kunststoffträgers, einer gelochten Membran, einer rechenartigen Anordnung von Säulen oder eines Metallgitters vor. Beispielsweise kann das Filterelement auch als Siliziumbauteil, z. B. mit einer gelochten Membran oder einem durch kleine Säulen rechenartig unterbrochenen Kanalstück, ausgeführt sein. Die Löcher, Poren oder Säulen weisen dabei bevorzugt eine Weite bzw. einen Abstand von kleiner als 100 Mikrometer auf.

Fig. 2 zeigt eine Schnittdarstellung des in Fig. 1 anhand eines Blockschaltbilds vorgestellten mikrofluidischen Systems 100, gemäß einem Ausführungsbeispiel der vorliegenden Erfindung. Wie die Darstellung in Fig. 2 zeigt, ist das beispielhafte mikrofluidische System 100 als ein polymerer Mehrschichtaufbau realisiert und besteht aus einem Bodenelement 200 sowie einem Deckelelement 202, wobei das Bodenelement 200 eine größere Dicke als das Deckelelement 202 aufweist. Die Kammer 102 ist in dem Bodenelement 200 angelegt und weist an einem Ende eine Verjüngung 204 auf, die bewirkt, dass Flüssigkeiten restlos aus der Kammer 102 entnommen werden können. Dies wird erreicht, indem sich die zweite Öffnung am bzgl. der Gravitation untersten Ende der Verjüngung befindet. Das Filterelement 106 ist im Deckelelement 202 direkt gegenüber der Verjüngung 204 angeordnet. Der anhand des Blockschaltbilds in Fig. 1 gezeigte Verbindungskanal entfällt somit bei dem in Fig. 2 gezeigten Ausführungsbeispiel des mikrofluidischen Systems 100, was den Vorteil hat, dass ein Totvolumen reduziert und eine Problematik eines Verstopfens des Verbindungskanals vermieden wird. Das Filterelement 106 ist bei dem in Fig. 2 gezeigten Ausführungsbeispiel in das Deckelelement 202 eingepresst, um eine seitliche Abdichtung des Filterelements 106 zu erreichen. Die Abmessungen der Kammer sind in jeder Raumrichtung vorteilhafterweise größer als ca. 3 Millimeter. Dies hat den Vorteil, dass in der Kammer vorhandene bzw. beim Einbringen von Flüssigkeiten in die Kammer entstehende Luftblasen nach oben steigen und aus dem System entfernt werden.

In der Darstellung in Fig. 2 ist das mikrofluidische Schichtsystem 100 in einer Positionierung im Betrieb und damit auf eine Seitenfläche aufgestellt gezeigt, d. h., das Bodenelement 200 befindet sich jetzt seitlich des Deckelelements 202. Wie die Darstellung in Fig. 2 zeigt, ist die Kammer 102 in Form einer Wanne in dem Bodenelement 200 angelegt. Die Verjüngung 204 ist durch Anschrägungen im Material des Bodenelements 200 an einem Ende der Wanne herausgebildet, wobei die Anschrägungen so verlaufen, dass in der in Fig. 2 gezeigten Position des mikrofluidischen Systems 100 jegliches in die Kammer 102 eingebrachte bewegliche Material, z. B. die in einer Analyse verwendeten Phasen, sich in dem verjüngten Abschnitt 204 gegenüber dem Filter 106 sammelt. Der Filter 106 ist bei dem in Fig. 2 gezeigten Ausführungsbeispiel des mikrofluidischen Systems 100 an einer der Kammer 102 zugewandten Hauptseite 206 des Deckelelements 202 angeordnet.

Der erste Kanal 112 zum Beschicken der Kammer 102 mit einer zu analysierenden Probe bildet eine erste Öffnung des mikrofluidischen Systems 100. Der an den Filter 106 angrenzende zweite Kanal 108 durchgreift das Deckelelement 202 weist eine zweite Öffnung 208 auf, über die Flüssigkeiten aus dem mikrofluidischen System 100 entnommen werden können. Der zweite Kanal oder Auslasskanal 108 erstreckt sich im Wesentlichen parallel zu der Verjüngung 204 in der Darstellung nach unten, sodass zu untersuchende Substanzen leicht aus dem mikrofluidischen System 100 entnommen werden können.

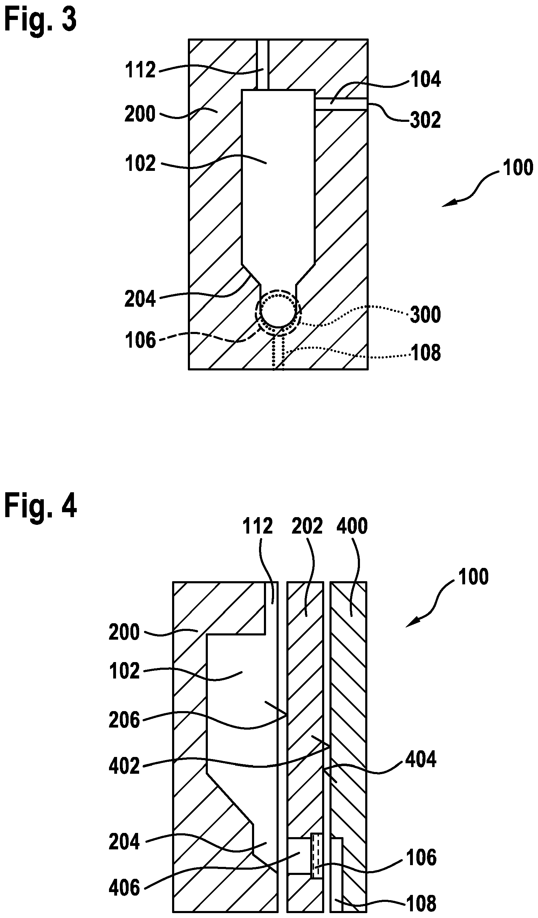

Fig. 3 zeigt das beispielhafte mikrofluidische System 100 aus Fig. 2 anhand eines Längsschnitts durch das Bodenelement 200. Ein Querschnitt des in dieser Ansicht dem verjüngten Abschnitt 204 der Kammer 102 nachgeschalteten Filters 106 ist mittels einer kreisrunden Strichlinie angedeutet. Der in dieser Ansicht dem Filter 106 nachgeschaltete zweite Kanal 108 ist mittels einer Punktlinie angedeutet. Ein Querschnitt einer an das Filterelement 106 angrenzenden Mündung 300 des zweiten Kanals 108 ist mittels einer kreisrunden Strichlinie angedeutet. Damit ist aus der Darstellung in Fig. 3 gut ersichtlich, dass ein Durchmesser des Filterelements 106 etwas größer, z. B. 0,5 bis 2 Millimeter größer, als eine Breite der Verjüngung 204 ist. Diese Maßnahme, den Filter 106 breiter als die Verjüngung 204 zu gestalten, gewährleistet eine bessere und gleichmäßigere Anströmung des Filters 106 sowie eine bessere Abdichtung des Übergangs zwischen Kammer 102 und Filter 106. Die Rückseite des Filterelements 106 wird durch den zweiten Kanal bzw. Ablaufkanal 108 kontaktiert.

Aus der Längsschnittdarstellung des beispielhaften mikrofluidischen Systems 100 in Fig. 3 ist gut ersichtlich, dass der das Bodenelement 200 quer durchgreifende erste Kanal 104 eine erste Öffnung 302 zum Einlassen einer Probe und/oder einer Mehrzahl von Mikropartikeln in die Kammer 102 aufweist. Ferner ist aus der Darstellung in Fig. 3 gut ersichtlich, dass bei dem gezeigten Ausführungsbeispiel des mikrofluidischen Systems 100 der verjüngte Abschnitt 204 eine maximale Entfernung von der ersten Öffnung 302 aufweist.

Fig. 4 zeigt wiederum in einer Schnittdarstellung ein weiteres Ausführungsbeispiel des mikrofluidischen Systems 100. Bei diesem Ausführungsbeispiel ist das Schichtsystem aus Bodenelement 200 und Deckelelement 202 um ein Abdeckelement 400 erweitert. Das Abdeckelement 400 weist einem dem Deckelelement 202 entsprechende Dicke auf und ist auf dem Deckelelement 202 angeordnet. Insbesondere ist bei dem in Fig. 4 gezeigten Ausführungsbeispiel der zweite Kanal 108 in dem Abdeckelement 400 angelegt, und zwar an einer dem Deckelelement 202 zugewandten Hauptseite 402 des Abdeckelements 400. Das Filterelement 106 ist im Gegensatz zum in den Figuren 2 und 3 vorgestellten Ausführungsbeispiel in einer der Hauptseite 206 gegenüberliegenden, von dem Bodenelement 200 mit der Kammer 102 abgewandten weiteren Hauptseite 404 des Deckelelements 202 angeordnet und über einen das Deckelelement 202 quer durchgreifenden Verbindungskanal 406 mit der Kammer 102 gekoppelt.

Die in Fig. 4 gezeigte alternative Ausführung des mikrofluidischen Systems 100, bei der das Filterelement 106 auf der Rückseite 404 des Deckelelements 202 positioniert ist, bietet den Vorteil, dass eine homogenere Anströmung des Filterelements 106 erreicht wird und das Filterelement 106 seitlich noch besser abgedichtet wird. Der weitere Deckel bzw. das Abdeckelement 400 dichtet den Aufbau aus Bodenelement 200 und Deckelelement 202 ab.

Gemäß weiteren Ausführungsbeispielen, die nicht in den Figuren gezeigt sind, ist die Kammer 102 in eine mit der ersten Öffnung 302 gekoppelte Lysekammer und eine den verjüngten Abschnitt 204 aufweisende Filterkammer unterteilt. Für die fluidische Koppelung können die Lysekammer und die Filterkammer über einen Durchtrittskanal verbunden sein. Diese Ausführung hat den Vorteil, dass das Filterelement 106 auch in größerer Entfernung von der Lysekammer positioniert werden kann. Weiterhin kann die Kammer 102 als Röhrchen ausgeführt sein und in ihrer Haupterstreckung quer zu der Hauptseite 206 des Deckelelements 202 in dem Bodenelement 200 angelegt sein. Das Filterelement 106 kann dann einen Boden der Kammer 102 bilden. Diese beispielhafte Ausführung hat den Vorteil, dass eine homogenere Anströmung des Filterelements 106 erreicht wird. Das Röhrchen kann z. B. auf den Mehrschichtaufbau aufgeklebt oder -geschweißt werden.

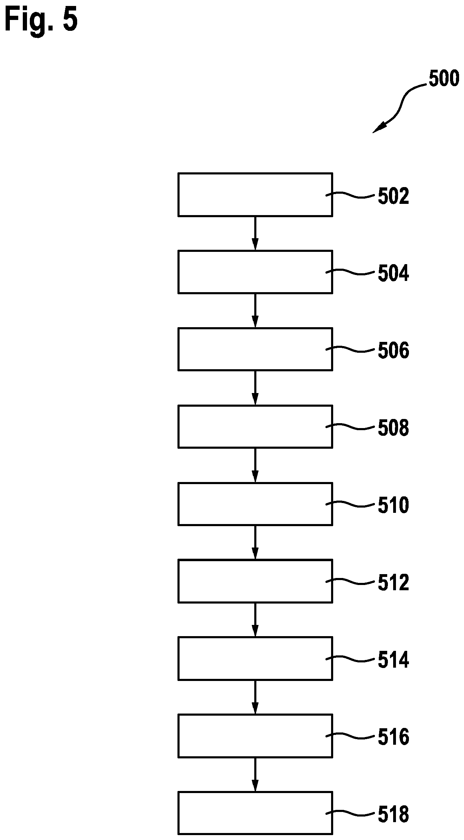

Fig. 5 zeigt ein Ablaufdiagramm eines Ausführungsbeispiels eines Verfahrens 500 zum Aufbereiten von organischen Zellen aus einer Probe. Das Verfahren 500 kann einem anhand der Figuren 1 bis 4 vorgestellten mikrofluidischen System ausgeführt werden.

In einem Schritt 502 werden über eine Einlassöffnung eines das Verfahren 500 ausführenden mikrofluidischen Systems Mikropartikel, beispielsweise kleine Kugeln mit einem Durchmesser im Mikrometerbereich, sogenannte Beads, in eine Kammer des mikrofluidischen Systems eingefüllt bzw. eingespült und in einem verjüngten Abschnitt der Kammer vor einem für die Mikropartikel und organische Zellen undurchlässigen Filter gepackt.

In einem Schritt 504 wird das mikrofluidische System mit der Mehrzahl von in dem verjüngten Abschnitt der Kammer gepackten Mikropartikeln bereitgestellt, sodass in einem Schritt 506 eine Probe mit einer Mehrzahl von organischen Zellen über die erste Öffnung in die Kammer des mikrofluidischen Systems eingelassen werden kann.

In einem Schritt 508 werden die organischen Zellen in dem verjüngten Abschnitt auf den dort gepackten Mikropartikeln akkumuliert. Unter der die organischen Zellen aufweisenden Probe kann eine die organischen Zellen enthaltende mobile Phase verstanden werden. Unter den Mikropartikeln kann eine stationäre Phase des das Verfahren ausführenden mikrofluidischen Systems verstanden werden. Die stationäre Phase ist ausgebildet, um in Wechselwirkung mit der mobilen Phase des mikrofluidischen Systems zu treten, um die organischen Zellen für eine nachfolgende Analyse aufzubereiten, beispielsweise, indem, gegebenenfalls in Kombination mit dem Lysemittel, eine in den organischen Zellen enthaltene DNA oder RNA aus den Zellen lysiert wird. Während des Akkumulierens werden die Mikropartikel durch die herrschende Strömung kontinuierlich gegen den Filter gedrückt, wodurch eine gleichbleibende Packungsdichte aufrechterhalten bzw. die Packungsdichte sogar noch weiter gesteigert werden kann. Hierdurch wird die Effizienz der Akkumulation weiter verbessert.

Die Schritte des Einlassens 506 und des Akkumulierens 508 können auch gleichzeitig stattfinden, indem die Probe kontinuierlich von der ersten Öffnung der Kammer her über die Mikropartikel gespült wird.

Gemäß einem Ausführungsbeispiel des Verfahrens 500 können die Schritte 502 bis 508 gleichzeitig oder in veränderter Reihenfolge ausgeführt werden. Beispielsweise werden die Mikropartikel zu der Probe hinzugegeben und gemeinsam mit dieser in das System eingespült, so dass gleichzeitig eine Packung der Mikropartikel und eine Akkumulation von organischen Zellen auf dieser Packung erfolgt. Dies hat den Vorteil, dass Prozessschritte und Flüssigkeiten eingespart werden und das Verfahren bzw. System schneller und platzsparender umgesetzt werden kann. Gemäß einem weiteren Ausführungsbeispiel kann das Verfahren 500 mit dem Schritt des Bereitstellens des die Mehrzahl von Mikropartikeln aufweisenden mikrofluidischen Systems beginnen. Dies bedeutet, dass beispielsweise die Mikropartikel bereits in einer Fertigung in das System eingefüllt werden.

In einem Schritt 510 wird über eine erste Öffnung des mikrofluidischen Systems ein Reinigungsmittel in die Kammer eingebracht, um die Mikropartikel und die organischen Zellen zu reinigen. Der Schritt 510 ist optional und kann gemäß Ausführungsbeispielen an weiteren Stellen des Verfahrens 500 wiederholt werden. In einem Schritt 512 des Verfahrens 500 wird ein Lysemittel in die Kammer eingespült, um eine Aufschließung der organischen Zellen und Freisetzung von in den Zellen enthaltenen Nukleinsäuren zu erreichen. Insbesondere wird in dem Schritt 512 das Lysemittel über die dem Filter nachgeschaltete zweite Öffnung des mikrofluidischen Systems in die Kammer eingespült, wodurch die Mikropartikel und organischen Zellen resuspendiert werden. Zum Binden der basierend auf der Aufschließung aus den Zellen freigesetzten Nukleinsäuren an die Mikropartikel wird in einem Schritt 514 ein Bindemittel in die Kammer eingebracht. Ein Einbringen des Bindemittels in die Kammer über die dem Filter nachgeschaltete zweite Öffnung ermöglicht hierbei eine besonders gute Durchmischung mit der bereits in der Kammer vorhandenen Flüssigkeit und ein effizientes Umspülen der Mikropartikel. An den Schritt 514 kann sich optional ein Schritt des Waschens der Mikropartikel und der an die Mikropartikel adsorbierten Nukleinsäuren anschließen.

In einem Schritt 516 werden die Mikropartikel bzw. Beads mit den an die Mikropartikel angebundenen Nukleinsäuren in dem verjüngten Abschnitt der Kammer gepackt. In einem Schritt 518 werden die Nukleinsäuren von den Mikropartikeln eluiert und für eine folgende Analyse durch den Filter und die zweite Öffnung aus dem mikrofluidischen System transportiert.

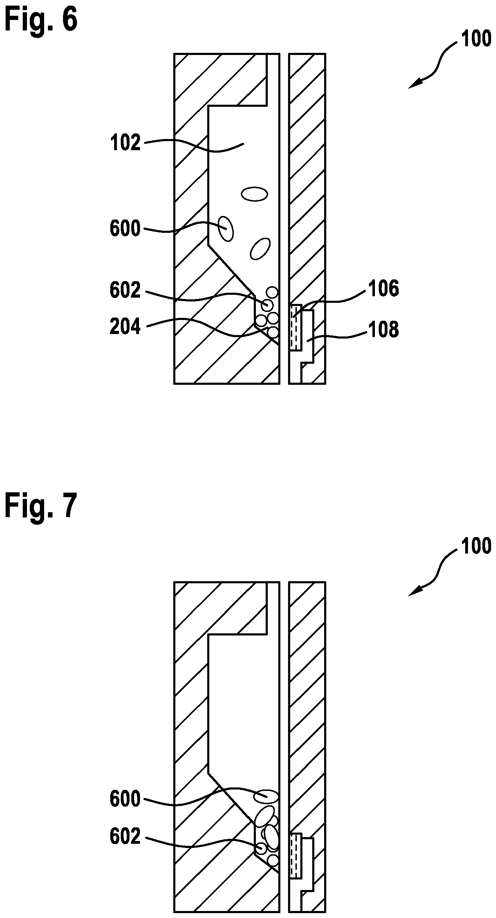

Fig. 6 bis 11 zeigen Phasenbilder eines Ausführungsbeispiels des anhand der Fig. 5 vorgestellten Verfahrens zum Aufbereiten von organischen Zellen aus einer Probe zur anschaulichen Erläuterung einer Funktionsweise des das Verfahren ausführenden mikrofluidischen Systems 100.

Fig. 6 veranschaulicht den Verfahrensschritt des Einlassens einer Probe, die eine Mehrzahl von organischen Zellen 600 aufweist, über die erste Öffnung (die durch den Kanal am oberen Ende der Kammer gebildet ist) des mikrofluidischen Systems 100 in die Kammer 102 bzw. eine frühe Phase im Schritt der Akkumulation der organischen Zellen, bei der sich noch Zellen im Bereich vor den Mikropartikeln befinden. Die Probe kann ein Volumen zwischen 50 Mikrolitern und 20 Millilitern aufweisen. Bei der Probe kann es sich um ein Körperfluid wie z. B. Blut, Urin, Sputum, um eine Lavage, einen ausgespülten Abstrich bzw. Swab oder eine verflüssigte Gewebeprobe oder auch um eine Zellsuspension aus einer Kultur handeln. Im Schritt des Einlassens wird die Probe mit den organischen Zellen 600 durch den Einlasskanal und die Kammer 102 in Richtung des Auslasskanals 108 gepumpt oder gesaugt. Hierfür können z. B. peristaltische oder Membranpumpen verwendet werden. Auch kann das Spülen manuell z.B. durch Pipettieren oder mit einer Spritze erfolgen. Insbesondere kann das dargestellte System Teil eines größeren mikrofluidischen Systems sein, das auch mikrofluidische Pumpen, z. B. eine mikrofluidische Membranpumpe, sowie Kammern, z. B. zum Probeneintrag, aufweist. Die Pumpe kann sich stromauf- oder stromabwärts der Kammer befinden.

Mikropartikel bzw. Beads 602 sind bereits in die Kammer 102 eingebracht und vor dem Filterelement 106 in dem verjüngten Abschnitt 204 der Kammer 102 zu einer Schüttung gepackt. Dieses Packen der Schüttung kann gemäß Ausführungsbeispielen bereits während der Herstellung des Systems 100 oder kurz vor einer Analyse bzw. einem Einlassen einer Probe geschehen, z. B. durch Einspülen der Mikropartikel 602, oder während der Analyse, z. B. indem sich die Mikropartikel 602 während des Einlassens der Probe zu einer Packung zusammenschieben. Das Filterelement 106 ist so ausgelegt, dass die Mikropartikel 602 zurückgehalten werden. Die Mikropartikel 602 können z. B. in Form von Silikakügelchen oder -teilchen mit einem Durchmesser zwischen 10 Mikrometern und 1 Millimeter vorliegen. Vorteilhafterweise überdecken die gepackten Mikropartikel das Filterelement vollständig. Hierdurch wird vermieden, dass ein Bypass an den gepackten Mikropartikeln vorbei entsteht, über den organische Zellen verloren gehen könnten.

Fig. 7 zeigt anhand eines weiteren Phasenbildes, wie die in der Probe enthaltenen Zellen 600, z. B. Humanzellen, Bakterien oder Pilze, auf der Schüttung aus Mikropartikeln 602 zurückgehalten und somit akkumuliert werden. Gezeigt ist der Zustand, in dem sich die Zellen 600 auf der Schüttung befinden, z. B. gegen Ende der Akkumulation.

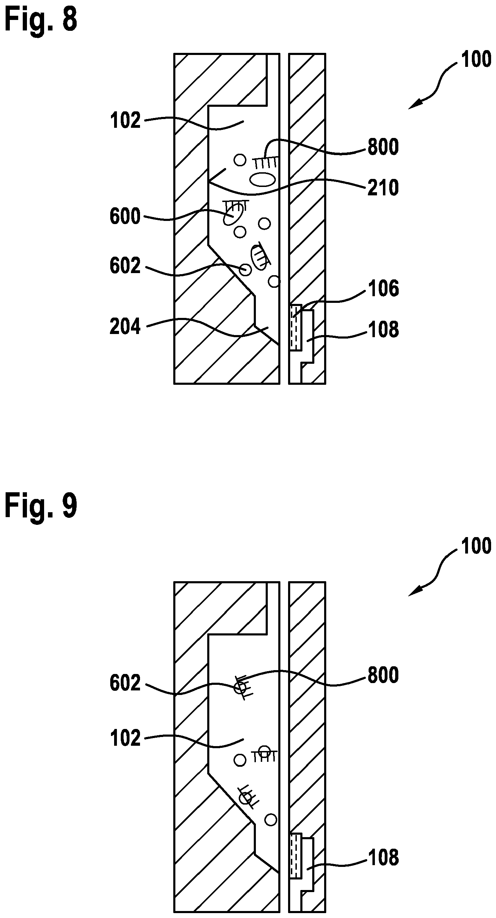

Fig. 8 zeigt ein Phasenbild des Verfahrensabschnitts der Resuspendierung der organischen Zellen 600 und Mikropartikel 602. Wie die Darstellung in Fig. 8 zeigt, werden die Mikropartikel 602 und akkumulierten Zellen 602 mittels eines Lysemittels bzw. Lysepuffers aus dem verjüngten Abschnitt 204 heraus in das Kammervolumen 102 hinein resuspendiert bzw. gewirbelt. Im in Fig. 8 gezeigten Ausführungsbeispiel wird die Resuspendierung erzielt, indem der Lysepuffer vom Auslasskanal 108 her in die Kammer 102 gepumpt wird. Die Resuspendierung kann jedoch prinzipiell auch durch Einspülen des Lysepuffers durch einen weiteren Kanal, der in den Bereich der Kammer, in dem sich die Mikropartikel befinden, mündet, erreicht werden. In der Kammer enthaltene Luft entweicht dabei über den Einlass- oder den Belüftungskanal. Der Lysepuffer bleibt über eine Inkubationszeit, z. B. zwischen 1 und 30 Minuten, in Kontakt mit den Zellen und bewirkt einen Aufschluss der Zellen 600, sodass in den Zellen 600 enthaltene Nukleinsäuren 800, z. B. DNA, freigesetzt werden. Der Lysepuffer kann hierzu Enzyme, z. B. Lysozym und/oder Proteinasen, und/oder chemische Lysereagenzien, z. B. Detergenzien oder chaotrope oder basische Komponenten, enthalten. Das Resuspendieren der Mikropartikel bewirkt hierbei, dass eine gute Durchmischung von Zellen und Lysepuffer erfolgt, eine homogene Konzentrationsverteilung des Lysemittels, z. B. der Enzyme, in der Kammer erreicht wird und der Lysepuffer sämtliche Zellen erreicht. Zusätzlich unterstützt ein Resuspendieren der Mikropartikel die Lyse durch ein Ausüben mechanischer Kräfte auf die Zellen. Die Resuspendierung der Mikropartikel erfolgt im einfachsten Fall durch Strömungskräfte.

Während der Lyse kann die Kammer beheizt werden, z.B. auf Temperaturen zwischen 30 und 60 °C. Dies hat den Vorteil, dass allgemein chemische Reaktionen schneller ablaufen und insbesondere ggf. für die Lyse verwendete Enzyme eine höhere Aktivität aufweisen. Auch ein Aufheizen der Kammer auf höhere Temperaturen, z.B. zwischen 90 und 97 °C, kann sinnvoll sein. In diesem Fall findet im Wesentlichen eine thermische Lyse statt.

Die Bewegung der Mikropartikel kann gemäß eines Ausführungsbeispiels während der Lyse in Gang gehalten werden, indem kontinuierlich oder in Schüben Luft vom Auslasskanal 108 her in die Kammer 102 gepumpt wird. Hierdurch kann eine kontinuierliche Agitation der Mikropartikel erreicht werden. Dies hat den Vorteil, dass während der gesamten Inkubationszeit mechanische Kräfte auf die organischen Zellen ausgeübt werden. Leicht zu lysierende Zellen können so gegebenenfalls alleine schon durch mechanische Kräfte lysiert werden.

Gemäß weiteren Ausführungsbeispielen kann das Resuspendieren der Mikropartikel 602 und Zellen 600 durch Stoßwellen oder Ultraschallpulse unterstützt werden. In diesem Fall ist eine Wandung 210 der Kammer 102 ganz oder teilweise als Membran ausgeführt, durch die die Stoßwellen oder die Ultraschallpulse eingekoppelt werden. Hierzu wird die Membran mit einem Injektor oder Ultraschallhorn in Kontakt gebracht. Dies hat außerdem den Vorteil, dass durch die direkte mechanische Agitation der Mikropartikel 602, gegebenenfalls auch durch Kavitation, eine noch stärkere Mahl- und damit Lysewirkung auf die Zellen 600 erzielt wird. Gegebenenfalls kann damit sogar vollständig auf die Zugabe z. B. von Enzymen oder chemischen Lysereagenzien in dem Lysepuffer verzichtet und als Lysemittel im einfachsten Fall Wasser oder ein wässriger Puffer verwendet werden. Eine während dieses Prozessschritts entstehende Flüssigkeit wird Lysat genannt.

Das in Fig. 9 gezeigte Phasenbild veranschaulicht den Verfahrensschritt des Einbringens eines Bindemittels bzw. Bindepuffers, das bzw. der vom Auslasskanal 108 her in die Kammer 102 eingespült wird. Das Lysat wird hierbei nicht aus der Kammer 102 verdrängt, sondern vermischt sich mit dem Bindepuffer. Hierdurch wird vermieden, dass Lysat und damit darin enthaltene Nukleinsäuren verloren gehen und für die Analyse nicht mehr zur Verfügung stehen. In einer Variante kann der Bindepuffer wiederum durch einen dritten Kanal in die Kammer gespült werden, wobei der Auslasskanal geschlossen bleibt, so dass kein Lysat entweicht. Der Bindepuffer bewirkt ein Binden der aus den Zellen herausgelösten Nukleinsäuren 800 an die Mikropartikel 602. Der Bindepuffer kann hierzu Alkohole, z. B. Ethanol oder Isopropanol, enthalten. Die Vermischung des Bindepuffers mit dem Lysat kann unterstützt werden, indem zusätzlich Luft durch den Auslasskanal in die Kammer gespült wird.

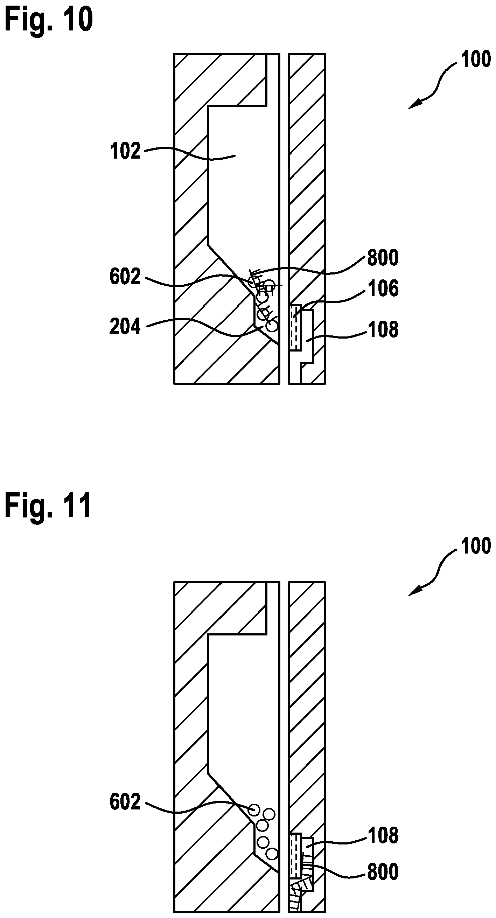

Fig. 10 zeigt ein Phasenbild zur Illustration einer Akkumulation der Mikropartikel 602 mit den an diese angebundenen Nukleinsäuren 800. Die Darstellung zeigt, wie die Mikropartikel 602 und Nukleinsäuren 800 aufweisende Mischung bzw. das mit Bindepuffer vermischte Lysat aus der Kammer 102 in Richtung des Auslasskanals 108 gespült wird. Die Mikropartikel 602 mit den adsorbierten Nukleinsäuren 800 packen sich hierbei erneut in dem verjüngten Abschnitt 204 der Kammer 102 vor dem Filterelement 106 zu einer Schüttung. In diesem Zustand können die Mikropartikel 602 gemäß Ausführungsbeispielen mit einem Waschmittel oder Waschpuffer gewaschen werden, um Zellreste, z. B. Proteine, zu entfernen. Hierzu kann z. B. ein Waschpuffer über den Einlasskanal in die Kammer hinein- und über den Auslasskanal aus dieser herausgepumpt werden. Alternativ kann ein Waschpuffer über den Auslasskanal in die Kammer gepumpt und anschließend über den Auslasskanal wieder abgesaugt werden. Dies hat den Vorteil, dass die Mikropartikel mit den adsorbierten Nukleinsäuren erneut resuspendiert und damit noch effizienter gewaschen werden können. Das Waschen der Mikropartikel kann auch mehrfach durchgeführt werden.

Fig. 11 veranschaulicht anhand eines weiteren Phasenbildes den erfindungsgemäßen Schritt des Eluierens und Transportierens der aus den Zellen herausgelösten Nukleinsäuren 800. Die Nukleinsäuren 800 werden von den Mikropartikeln bzw. Kügelchen 602 eluiert, indem Wasser oder ein Elutionspuffer bzw. -mittel über die Schüttung in den Auslasskanal 108 gespült wird.

In einer Variante des anhand der in den Figuren 6 bis 11 gezeigten Phasenbilder vorgestellten Verfahrens zum Aufbereiten von organischen Zellen aus einer Probe entfallen die anhand der Figuren 9, 10 und 11 erläuterten Verfahrensschritte. Das Lysat, gegebenenfalls einschließlich der Mikropartikel, wird dann zunächst aus der Kammer 102 entnommen, und die Nukleinsäuren 800 werden aufgereinigt, indem sie z. B. an eine Membran, z. B. eine Silikamembran, gebunden werden. Dies hat den Vorteil, dass auch Mikropartikel 602 verwendet werden können, die nicht zur Bindung von Nukleinsäuren 800 geeignet sind. Die Entnahme des Lysats aus der Kammer kann z. B. durch Pipettieren erfolgen oder Spülen durch einen weiteren Kanal, der vor dem Filter in die Kammer mündet.

In einer weiteren Variante des hier vorgestellten Verfahrens wird nach dem anhand der Darstellung in Fig. 8 illustrierten Verfahrensschritt der Resuspendierung noch ein Verdau von im Lysat enthaltenen Proteinen durchgeführt. Hierzu wird ein Puffer, der z. B. Proteinase enthält, durch den Auslasskanal 108 in die Kammer 102 gespült, dort mit dem Lysat vermischt und inkubiert. Dies hat den Vorteil, dass Verunreinigungen, z. B. Proteine, noch effektiver entfernt werden.

Gemäß einer weiteren Variante des Verfahrens können in den Schritten des Bindens der Nukleinsäuren an die Mikropartikel, des Waschens der an die Mikropartikel adsorbierten Nukleinsäuren und des Eluierens jeweils kurz Stoßwellen oder Ultraschallenergie in die Kammer eingekoppelt werden. Dies kann den Vorteil haben, dass in diesen Schritten die Resuspendierung der Beads verbessert und so die Effizienz der Bindung bzw. Waschung bzw. Elution verbessert wird.

Weiterhin können die Mikropartikel und der Filter vor dem Schritt des Eluierens der Nukleinsäuren getrocknet werden.

Weiterhin können gemäß einem Ausführungsbeispiel des hier vorgestellten Verfahrens nach dem anhand der Darstellung in Fig. 7 illustrierten Verfahrensschritt des Akkumulierens der Zellen 600 auf der Schüttung aus Mikropartikeln 602 die akkumulierten Zellen 600 gewaschen werden, z. B. mit einem Waschpuffer bzw. -mittel. Diese Ausführung des Verfahrens hat den Vorteil, dass Bestandteile der Probe entfernt werden können, die die weitere Aufreinigung und Detektion stören würden.

Weiterhin kann vor Schritt I eine vorhergehende Aufbereitung der Probe erfolgen.

Z. B. können in der Probe enthaltene humane Zellen, z. B. Blutzellen, durch Zugabe von Proteinasen oder Detergenzien oder durch osmotischen Schock oder kurzes Aufheizen, z. B. auf 70 °C, lysiert werden. In der Probe enthaltene Bakterien oder Pilze bleiben dabei intakt und können in den darauffolgenden Schritten auf der Schüttung akkumuliert werden. Auch kann als vorhergehende Aufbereitung eine Verflüssigung von Gewebebestandteilen und ein Verdau von Proteinen erfolgen, z. B. durch Zugabe von Proteinase.

Gemäß einem weiteren Ausführungsbeispiel kann als vorhergehende Aufbereitung zu dem anhand des Phasenbildes in Fig. 6 veranschaulichten Verfahrensschritt des Einlassens der Probe in die Kammer 102 ein die Probe enthaltender Abstrich bzw. Swab ausgespült werden. Die vorhergehende Aufbereitung hat den Vorteil, dass die Zellen 600, deren Nukleinsäure detektiert werden soll, der Aufreinigung besser zugänglich gemacht werden kann.

In einer weiteren Variante können mit Silika beschichtete magnetische Mikropartikel verwendet werden. Diese können in den Schritten der Lyse, der Bindung der Nukleinsäuren an die Mikropartikel, des Waschens der an die Mikropartikel adsorbierten Nukleinsäuren und des Eluierens jeweils mittels eines externen Magnetfeldes in Bewegung versetzt werden. Hierdurch kann die Effizienz der Lyse bzw. Bindung bzw. Waschung bzw. Elution verbessert werden. Die Agitation der Mikropartikel mittels magnetischer Felder stellt eine Alternative zur Agitation der Mikropartikel mittels Strömungskräften, Stoßwellen oder Ultraschallpulsen dar. Die Verwendung magnetischer Felder kann hierbei gegenüber der Verwendung von Stoßwellen oder Ultraschallpulsen den Vorteil haben, dass kein mechanischer Zugang zu einer Außenfläche der Kammer nötig ist, wodurch ggf. Bauraum eingespart werden kann.

Das anhand der Phasenbilder in den Figuren 6 bis 11 vorgestellte Verfahren ermöglicht die Akkumulation von Pathogenen aus einer Probe, deren anschließende Lyse sowie die Aufreinigung der enthaltenen Nukleinsäure und zeichnet sich insbesondere dadurch aus, dass auch bei einer nur geringen Konzentration von Pathogenen in einer Probe eine aussagekräftige Analyse der Pathogene ermöglicht werden kann.

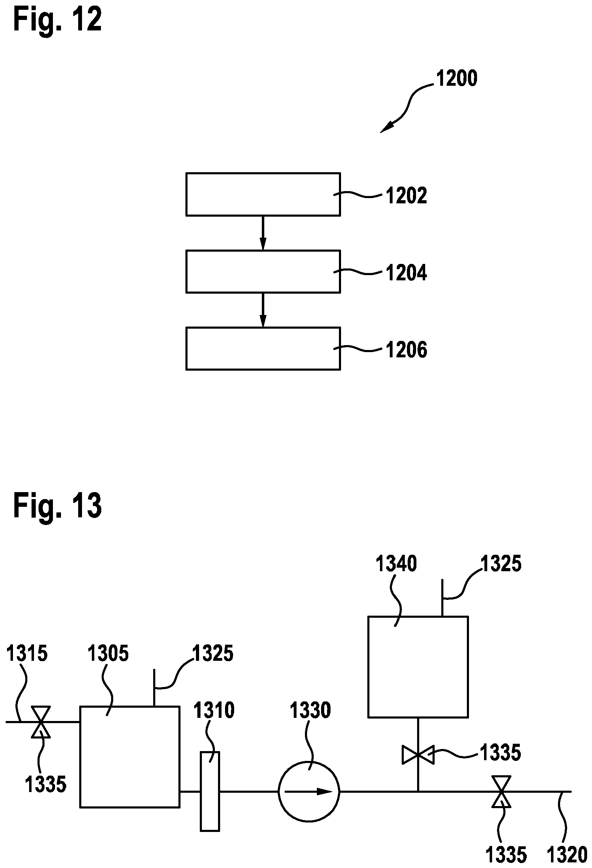

Fig. 12 zeigt ein Ablaufdiagramm eines Ausführungsbeispiels eines Herstellungsverfahrens 1200 zum Herstellen eines mikrofluidischen Systems zum Aufbereiten von organischen Zellen. Bei einem mittels des Herstellungsverfahrens 1200 hergestellten mikrofluidischen System kann es sich um ein Ausführungsbeispiel des anhand der Figuren 1 bis 4 vorgestellten mikrofluidischen Systems handeln.

In einem Schritt 1202 wird ein Bodenelement mit einer in dem Bodenelement wannenförmig angelegten Kammer und einem mit der Kammer gekoppelten und das Bodenelement durchgreifenden ersten Kanal bereitgestellt. In einem Schritt 1204 werden ein Deckelelement und ein Filter bereitgestellt. In einem Schritt 1206 wird das Deckelelement so auf das Bodenelement aufgesetzt, dass der Filter einem verjüngten Abschnitt der Kammer gegenüberliegend oder benachbart angeordnet wird.

Fig. 13 zeigt ein Blockschaltbild eines beispielhaften mikrofluidischen Systems zur Durchführung des erfindungsgemäßen Verfahrens. Neben der Kammer 1305 mit Filter 1310, Einlasskanal 1315, Auslasskanal 1320 und Belüftungskanal 1325 weist das System eine mikrofluidische Pumpe 1330, mikrofluidische Ventile 1335 sowie weitere Kammern 1340 (nur eine dargestellt) auf. Die Kammern 1340 dienen als Reservoire für die Bevorratung von Reagenzien, z. B. des Lysepuffers, des Bindepuffers, des Waschpuffers und/oder des Elutionspuffers. Weitere Reservoire, z. B. zur Bevorratung der Probe, von Flüssigkeiten zum Einspülen der Mikropartikel und weiterer Waschpuffer, können mit dem Einlasskanal 1315 verbunden sein. Die Kammern 1340 können mittels Be- oder Entlüftungskanälen 1325 ebenfalls belüftet sein, um einen Druckausgleich bei der Entnahme von Flüssigkeit aus den Kammern zu erreichen. Die Pumpe 1330 dient zum Spülen der Probe sowie gegebenenfalls von Waschpuffern über den Einlasskanal in den Auslasskanal. Weiterhin können mit der Pumpe 1330 Lyse-, Binde- und Waschpuffer vom Auslass her in die Kammer gespült und wieder abgesaugt werden.

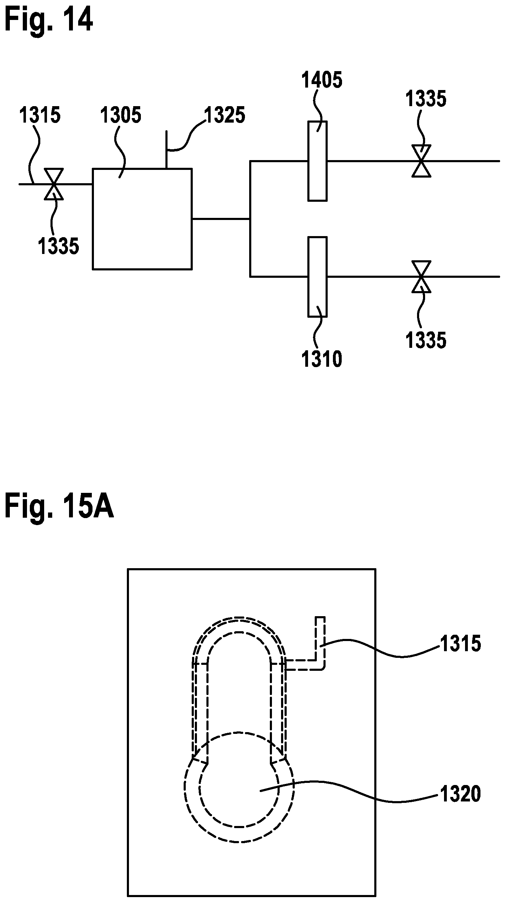

Fig. 14 zeigt ein Blockschaltbild einer Topologie von Kammer 1305, Filter 1310 und zweiter Öffnung, bei der parallel zum Filter 1310 eine Membran 1405 geschaltet ist, die zur Aufreinigung von Nukleinsäuren geeignet ist, z.B. eine Silikamembran. Nach der Lyse wird vom Pfad mit Filter 1310 auf den Pfad mit der Membran 1405 umgeschaltet. Beim Abpumpen des Bindepuffers/Waschpuffers zum Waschen der Nukleinsäuren/Elutionspuffers packen sich die Mikropartikel diesmal vor der Membran 1405. Hierdurch ergibt sich als Vorteil, dass die Nukleinsäuren noch effizienter aufgereinigt werden können, insbesondere bei einer hohen Last an Nukleinsäuren und/oder es können Mikropartikel verwendet werden, die nicht zur Aufreinigung von Nukleinsäuren geeignet sind. Verbindungskanäle zwischen Kammer und Filter/Membran können wieder entfallen, d. h., die Membran 1405 kann ähnlich wie Filter 1310 direkt an der Kammer 1305 angeordnet sein.

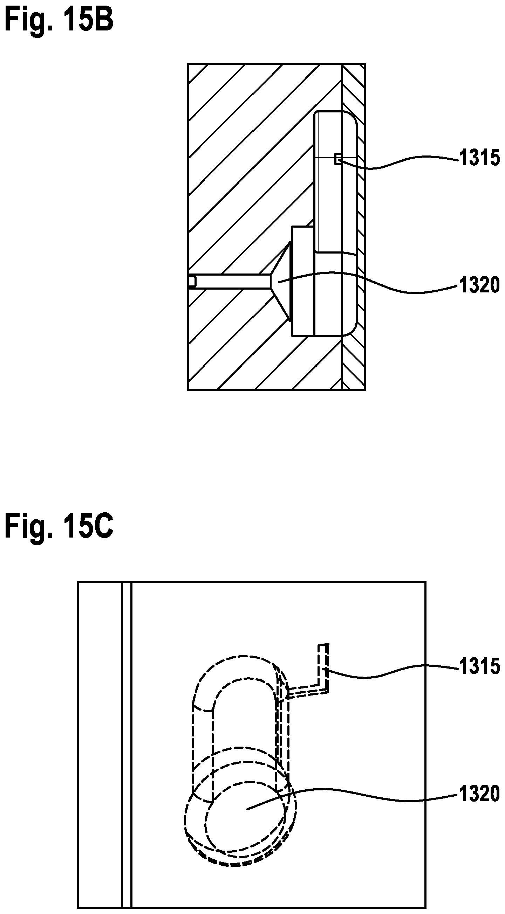

Die Teilfiguren der Fig. 15 zeigen einen Hauptteil der Kammer 1305 und des Filters 1310 in einer Schicht in Aufsichtsdarstellung (Fig. 15A), in Querschnittsdarstellung (Fig. 15B) und in perspektivischer Ansicht (Fig. 15C). Der Einlasskanal 1315 (wobei dessen weitere Führung nicht dargestellt ist) ist seitlich dargestellt und der Auslasskanal 1320 ist als Durchloch dargestellt. Die Verjüngung entfällt in dem in den Teilfiguren 15 gezeigten Ausführungsbeispiel. Ein Entlüftungskanal ist nicht dargestellt.

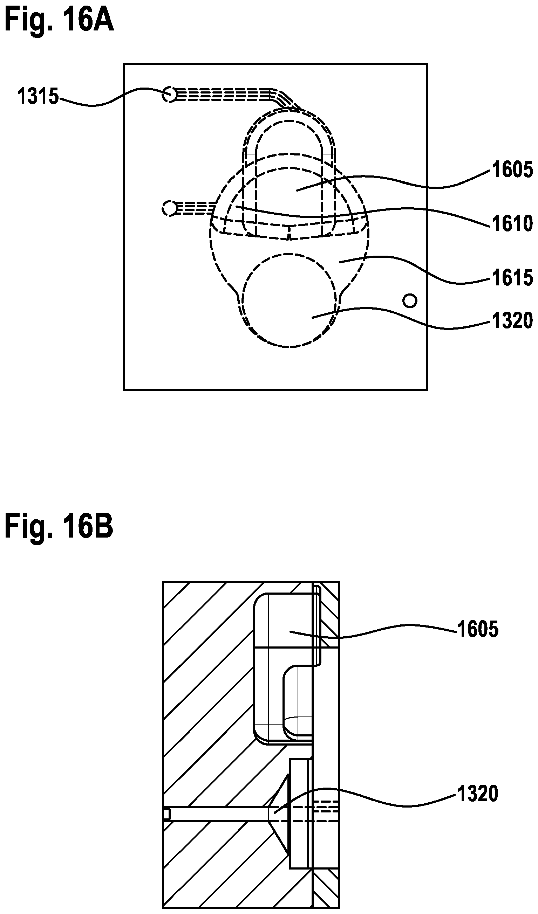

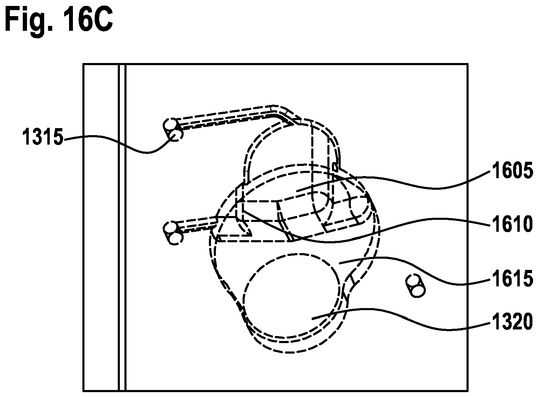

Die Teilfiguren der Fig. 16 zeigen eine weitere Darstellung eines Hauptteils der Kammer 1305 und des Filters1310 in unterschiedlichen Ansichten (Aufsichtdarstellung in Fig. 16A, Querschnittsdarstellung in Fig. 16B und perspektivische Darstellung in Fig. 16C). Hierbei ist eine zusätzliche Erweiterung 1605 und Durchbruch 1610 nach außen dargestellt. Der kreisförmige Durchbruch 1610 wird durch eine weitere Membran 1615 gedeckelt.

Das hierin vorgestellte Konzept zur Aufbereitung von organischen Zellen ist beispielsweise in Systemen oder Laborroutinen einsetzbar, die zur Diagnose von Infektionskrankheiten verwendet werden.

Die beschriebenen und in den Figuren gezeigten Ausführungsbeispiele sind nur beispielhaft gewählt. Unterschiedliche Ausführungsbeispiele können vollständig oder in Bezug auf einzelne Merkmale miteinander kombiniert werden. Auch kann ein Ausführungsbeispiel durch Merkmale eines weiteren Ausführungsbeispiels ergänzt werden.

Ferner können die hier vorgestellten Verfahrensschritte wiederholt sowie in einer anderen als in der beschriebenen Reihenfolge ausgeführt werden.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Verfahren (500) zum Aufbereiten von organischen Zellen (600), wobei das Verfahren (500) die folgenden Schritte aufweist:
Bereitstellen (504) eines mikrofluidischen Systems (100) mit einer eine stationäre Phase in Form einer Mehrzahl von Mikropartikeln (602) aufweisenden Kammer (102);
Einlassen (506) einer Mehrzahl von organischen Zellen (600) in einer mobilen Phase in die Kammer (102) über eine erste Öffnung (302) des mikrofluidischen Systems (100);
Akkumulieren (508) der organischen Zellen (600) in einem Abschnitt (204) der Kammer (102), in dem die Mikropartikel (602) aufgeschüttet sind und der einem für die Mikropartikel (602) undurchlässigen Filter (106) des mikrofluidischen Systems (100) vorgeschaltet ist; und
**gekennzeichnet durch** ein
Einspülen (512) eines Lysemittels in die Kammer (102) über einen Kanal, der in den Abschnitt (204) der Kammer (102) mündet, um für eine Aufschließung der organischen Zellen (600) die Mikropartikel (602) und die organischen Zellen (600) in der Kammer (102) zu resuspendieren.

2. Verfahren (500) gemäß Anspruch 1, **dadurch gekennzeichnet, dass** in dem Schritt des Einspülens (512) das Lysemittel über eine dem Filter (106) nachgeschaltete zweite Öffnung (208) des mikrofluidischen Systems (100) in die Kammer (102) eingespült wird.

3. Verfahren gemäß Anspruch 1 oder 2, **dadurch gekennzeichnet, dass** nach dem Einspülen des Lysemittels die Mikropartikel durch die Einkopplung von Ultraschall oder Stoßwellen in die Kammer in Bewegung versetzt werden.

4. Verfahren (500) gemäß einem der vorangegangenen Ansprüche, **dadurch gekennzeichnet, dass** das Verfahren (500) einen Schritt des Einfüllens (502) der Mikropartikel (602) über die erste Öffnung (302) in die Kammer (102) und/oder des Packens der Mikropartikel (602) in dem verjüngten Abschnitt (204) der Kammer (102) aufweist.

5. Verfahren (500) gemäß einem der vorangegangenen Ansprüche, **dadurch gekennzeichnet, dass** das Verfahren (500) einen Schritt des Einbringens (514) eines Bindemittels über die zweite Öffnung (208) in die Kammer (102) aufweist, um eine basierend auf der Aufschließung aus den Zellen (600) freigesetzte Nukleinsäure (800) an die Mikropartikel (602) zu binden.

6. Verfahren (500) gemäß einem der vorangegangenen Ansprüche, **dadurch gekennzeichnet, dass** das Verfahren (500) einen Schritt des Packens (516) der Mikropartikel (602) mit der an die Mikropartikel (602) angebundenen Nukleinsäuren (800) in dem verjüngten Abschnitt (204) der Kammer (102) aufweist.

7. Verfahren (500) gemäß einem der vorangegangenen Ansprüche, **dadurch gekennzeichnet, dass** das Verfahren (500) einen Schritt des Eluierens (518) der Nukleinsäuren (800) von den Mikropartikeln (602) und des Transportierens der Nukleinsäure (800) durch den Filter (106) und die zweite Öffnung (208) oder eine weitere Öffnung aus der Kammer (102) aufweist.

8. Verfahren (500) gemäß einem der vorangegangenen Ansprüche, **dadurch gekennzeichnet, dass** das Verfahren (500) einen Schritt des Einbringens (510) eines Reinigungsmittels durch die zweite Öffnung (208) in die Kammer (102) aufweist, um die Mikropartikel (602) mit den an die Mikropartikel (602) angebundenen Nukleinsäuren (800) zu reinigen.

9. Verfahren gemäß einem der vorhergehenden Ansprüche, **dadurch gekennzeichnet, dass** nach dem Schritt des Aufschließens der organischen Zellen die Aufreinigung der Nukleinsäuren auf einer separaten Membran durchgeführt wird.

## Claims

1. Method (500) for processing organic cells (600), wherein the method (500) comprises the following steps:
providing (504) a microfluidic system (100) having a chamber (102) comprising a stationary phase in the form of a plurality of microparticles (602);
letting (506) a plurality of organic cells (600) in a mobile phase into the chamber (102) across a first opening (302) of the microfluidic system (100) ;
accumulating (508) the organic cells (600) in a section (204) of the chamber (102) in which the microparticles (602) are deposited and that is upstream of a filter (106) of the microfluidic system (100) that is impermeable to the microparticles (602); and
**characterized by**
flushing (512) a lysis agent into the chamber (102) via a channel which opens into the section (204) of the chamber (102) in order to resuspend the microparticles (602) and the organic cells (600) in the chamber (102) for a disruption of the organic cells (600).

2. Method (500) according to Claim 1, **characterized in that,** in the step of flushing (512), the lysis agent is flushed into the chamber (102) across a second opening (208) of the microfluidic system (100) that is downstream of the filter (106).

3. Method according to Claim 1 or 2, **characterized in that**, after flushing in the lysis agent, the microparticles are set into motion by the coupling of ultrasound or shockwaves into the chamber.

4. Method (500) according to any of the preceding claims, **characterized in that** the method (500) comprises a step of filling (502) the microparticles (602) across the first opening (302) into the chamber (102) and/or of packing the microparticles (602) in the tapered section (204) of the chamber (102).

5. Method (500) according to any of the preceding claims, **characterized in that** the method (500) comprises a step of introducing (514) a binder across the second opening (208) into the chamber (102) in order for a nucleic acid (800) released from the cells (600) on the basis of the disruption to bind to the microparticles (602).

6. Method (500) according to any of the preceding claims, **characterized in that** the method (500) comprises a step of packing (516) the microparticles (602) having the nucleic acids (800) bound to the microparticles (602) in the tapered section (204) of the chamber (102).

7. Method (500) according to any of the preceding claims, **characterized in that** the method (500) comprises a step of eluting (518) the nucleic acids (800) from the microparticles (602) and of transporting the nucleic acid (800) from the chamber (102) through the filter (106) and the second opening (208) or a further opening.

8. Method (500) according to any of the preceding claims, **characterized in that** the method (500) comprises a step of introducing (510) a cleaning agent through the second opening (208) into the chamber (102) in order to clean the microparticles (602) having the nucleic acids (800) bound to the microparticles (602).

9. Method according to any of the preceding claims, **characterized in that**, after the step of disrupting the organic cells, the purification of the nucleic acids is carried out on a separate membrane.

## Revendications

1. Procédé (500) de traitement de cellules organiques (600), le procédé (500) présentant les étapes suivantes :
prévoir (504) un système microfluide (100) qui présente une phase stationnaire sous la forme de plusieurs chambres (102) dotées de microparticules (602),
introduire (506) plusieurs cellules organiques (600) placées dans une phase mobile dans une chambre (102) par une première ouverture (302) du système microfluide (100),
accumuler (508) les cellules organiques (600) dans une section (204) de la chambre (102) dans laquelle sont versées les microparticules (602) et située en amont d'un filtre (106) du système microfluide (100) imperméable aux microparticules (602),
**caractérisé par**
l'introduction (512) d'un agent de lyse dans la chambre (102) par un canal qui débouche dans la section (204) de la chambre (102) pour remettre en suspension les microparticules (602) et les cellules organiques (600) dans la chambre (102) en vue de désagréger les cellules organiques (600).

2. Procédé (500) selon la revendication 1, **caractérisé en ce que** dans l'étape d'introduction (512), l'agent de lyse est injecté dans la chambre (102) par l'intermédiaire d'une deuxième ouverture (208) du système microfluide (100) située en aval du filtre (106).

3. Procédé selon les revendications 1 ou 2, **caractérisé en ce qu'**après l'introduction de l'agent de lyse, les microparticules sont mises en déplacement dans la chambre par l'application d'ultrasons ou d'ondes de choc.

4. Procédé (500) selon l'une des revendications précédentes, **caractérisé en ce que** le procédé (500) présente une étape (502) d'introduction des microparticules (602) dans la chambre (102) par la première ouverture (302) et/ou d'empilement des microparticules (602) dans la partie rétrécie (204) de la chambre (102).

5. Procédé (500) selon l'une des revendications précédentes, **caractérisé en ce que** le procédé (500) présente une étape (514) d'apport d'un liant dans la chambre (102) par la deuxième ouverture (208) pour lier aux microparticules (602) un acide nucléique (800) libéré hors des cellules (600) suite à leur désagrégation.

6. Procédé (500) selon l'une des revendications précédentes, **caractérisé en ce que** le procédé (500) présente une étape (516) d'empilement des microparticules (602) avec les acides nucléiques (800) liés aux microparticules (602) dans la section rétrécie (204) de la chambre (102).

7. Procédé (500) selon l'une des revendications précédentes, **caractérisé en ce que** le procédé (500) présente une étape (518) de dilution des acides nucléiques (800) des microparticules (602) et de transport hors de la chambre (102) de l'acide nucléique (800) à travers le filtre (106) et la deuxième ouverture (208) ou une autre ouverture.

8. Procédé (500) selon l'une des revendications précédentes, **caractérisé en ce que** le procédé (500) présente une étape (510) d'apport d'un agent de nettoyage dans la chambre (102) par la deuxième ouverture (208) pour nettoyer des microparticules (602) avec les acides nucléiques (800) liés aux microparticules (602).

9. Procédé selon l'une des revendications précédentes, **caractérisé en ce qu'**après l'étape de désagrégation des cellules organiques, le nettoyage des acides nucléiques est réalisé sur une membrane séparée.
